(19) Europäisches Patentamt
European Patent Office
Office européen des brevets

(11) Numéro de publication : **0 442 769 A2**

(12) **DEMANDE DE BREVET EUROPEEN**

(21) Numéro de dépôt : **91400127.6**

(22) Date de dépôt : **21.01.91**

(51) Int. Cl.$^5$ : **C07D 489/08**, C07F 9/6561, A61K 31/485

(30) Priorité : **19.01.90 FR 9000613**

(43) Date de publication de la demande : **21.08.91 Bulletin 91/34**

(84) Etats contractants désignés : **AT BE CH DE DK ES FR GB GR IT LI LU NL SE**

(71) Demandeur : **FONDATION NATIONALE DE TRANSFUSION SANGUINE**
**6, rue Alexandre Cabanel**
**F-75739 Paris Cédex 15 (FR)**

(72) Inventeur : **Guillaumet, Gérald**
**2 rue A. Renoir**
**F-45100 Orléans (FR)**
Inventeur : **Ropars, Claude**
**16 rue de la Pinterie**
**F-37550 St Avertin (FR)**
Inventeur : **Meunier, Jean-Claude**
**8 Chemin du Pin, Rebigue**
**F-31320 Castanet Tolosan (FR)**

(74) Mandataire : **Warcoin, Jacques et al**
**Cabinet Régimbeau 26, avenue Kléber**
**F-75116 Paris (FR)**

(54) **Composés destinés à l'encapsulation dans les érythrocytes-nouveaux dérivés de la naloxone et naltrexone.**

(57) La présente invention se rapporte à un dérivé destiné à l'encapsulation dans les érythrocytes d'un composé biologiquement actif hydrophobe caractérisé en ce qu'il est constitué dudit composé biologiquement actif couplé chimiquement à un groupement chimique à caractère hydrophile, par un élément de liaison qui est clivé par les enzymes lysosomiales et/ou plasmatiques mais qui est stable dans les érythrocytes.

Elle concerne plus particulièrement les dérivés de formule I :

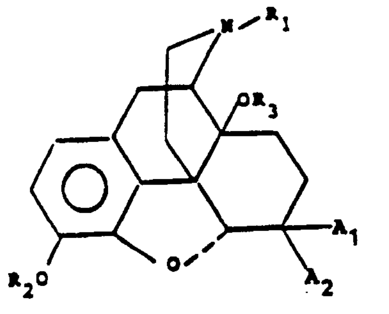

(I)

les compositions pharmaceutiques les contenant et les érythrocytes rescellés, de même que leur utilisation à titre de prodrogues de la naloxone ou de la naltrexone.

EP 0 442 769 A2

# COMPOSES DESTINES A L'ENCAPSULATION DANS LES ERYTHROCYTES - NOUVEAUX DERIVES DE LA NALOXONE ET NALTREXONE

Cette invention réalisée dans les Laboratoires suivants : Laboratoire de Chimie Bioorganique et Analytique - Université d'Orléans ; Laboratoire de Biopharmacologie transfusionnelle-CRTS de Tours ; Laboratoire de Pharmacologie et toxicologie fondamentales-CNRS de Tours, se rapporte à des composés chimiques utiles notamment pour l'internalisation érythrocytaire de composés biologiquement actifs et plus particulièrement à de nouveaux dérivés de la naloxone et de la naltrexone.

Il a déjà été réalisé des encapsulations de substances dans les érythrocytes (Green R. et Al, The LANCET (1980) ; p 327 ; ROPARS, NICOLAU, CHASSAIGNE - FR 82 11749)

Cependant, l'utilisation de globules rouges pour le transport de médicaments hydrophobes pose encore aujourd'hui des difficultés lors de l'interaction de ce type de médicaments avec la membrane érythrocytaire. On assiste soit à une fuite plus ou moins rapide du vecteur, soit à une adsorption considérable qui ne permet pas une internalisation du produit.

Un des objets de la présente invention est précisément de proposer une solution au problème précité.

Plus particulièrement la présente invention se rapporte à un dérivé destiné à l'encapsulation dans les érythrocytes d'un composé biologiquement actif hydrophobe caractérisé en ce qu'il est constitué dudit composé biologiquement actif couplé chimiquement à un groupement chimique à caractère hydrophile par un élément de liaison qui est clivé par les enzymes lysosomiales et/ou plasmatiques mais qui est stable dans les érythrocytes.

De tels composés chimiques de par leur structure présentent un caractère hydrophile accentué, une interaction faible avec la membrane érythrocytaire et une stabilité chimique à l'égard des diverses enzymes présentes dans ce type de cellules. Enfin, lors de la destruction des érythrocytes par le système phagocytaire, ils peuvent être clivés par les enzymes lysosomiales ou plasmatiques et libérer ainsi le médicament.

Le groupement chimique à caractère hydrophile utilisé selon l'invention peut être une chaîne contenant un ou plusieurs sucres et/ou un ou plusieurs acides aminés. Il peut également s'agir d'un radical sulfate phosphate, polyphosphate ou autre fonction hydrophile.

Parmi les composés chimiques objet de la présente invention on citera tout particulièrement de nouveaux dérivés de la naloxone, de la naltrexone, et du naltrexol.

La naloxone est un antagoniste pur et spécifique de morphinomimétiques sans effet agoniste.

La présente invention se rapporte donc également à un composé chimique selon l'invention caractérisé en ce qu'il est représenté par la formule générale I :

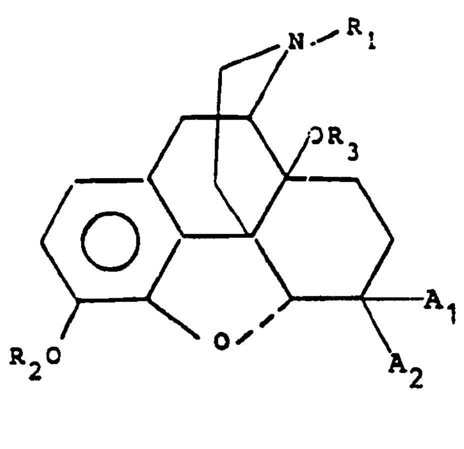

(I)

dans laquelle :

– soit $A_1$ représente le groupement OH et $A_2$ est l'atome d'hydrogène, soit $A_1$ et $A_2$ ensemble avec l'atome de carbone auxquels ils sont rattachés forment un groupement carbonyle

$$(>C=O),$$

– $R_1$ représente un groupement $-CH_2-CH=CH_2$ ou

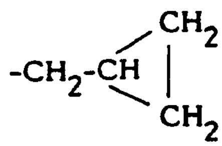

– **$OR_2$** représente un groupement sulfate ou un groupement phosphate ou polyphosphate de formule II :

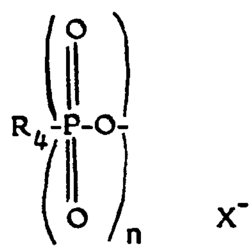

(II)

dans laquelle :

. $R_4$ est choisi parmi un alkoxy en $C_{1-3}$, OH et ses sels alcalins,

. X est un cation alcalin et de préférence $Na^+$, et

. n représente un entier de 1 à 3, et

– **$OR_3$** représente OH ou un groupement sulfate, à la condition que si $R_1$ représente $-CH_2-CH=CH_2$ et **A** un groupement CHOH, $OR_2$ et/ou $OR_3$ sont différents d'un groupement sulfate.

Les dérivés de la naloxone et de la naltrexone sont des esters de naloxone et de naltrexone obtenus par greffage sur l'hydroxyle en position 3 d'un groupement monophosphate ou polyphosphate ou, sur les hydroxyles en positions 3 et/ou 14, de groupements sulfate.

Parmi ces composés, on citera tout particulièrement les dérivés dans lesquels $OR_3$ représente un groupement OH et $OR_2$ un groupement phosphate ou triphosphate de formule II.

Ces composés de formule générale I sont intéressants à titre de prodrogues de la naloxone ou naltrexone. En raison de leur caractère hydrophile, ils peuvent être internalisés de manière satisfaisante dans les globules rouges. Enfin, lors de la destruction desdits globules rouges, ils sont hydrolysés in vivo et libèrent alors la molécule du composé biologiquement actif, c'est-à-dire la naloxone ou la naltrexone.

Ces composés sont obtenus par estérification d'au moins un hydroxyle de la naloxone ou de la naltrexone.

Alors que le disulfate de naloxone en position 3 et 14 et le monosulfate en position 3 ont déjà fait l'objet d'une description dans la littérature (Linder C. and Fishman J., J. Medicinal Chem. 16, 553, 1973), les différents sulfates de la naltrexone étaient aujourd'hui encore inconnus.

A partir des précédents travaux cités, les inventeurs ont donc après modification du protocole opératoire, isolé les deux dérivés de la naltrexone recherchés. L'obtention du disulfate fait intervenir une sulfatation directe, celle du monosulfate en position 3 un processus de blocage et de déblocage utilisant les esters acétiques.

Dans le cadre de la préparation des dérivés phosphatés de formule générale 1, le traitement de la naloxone ou naltrexone par le dérivé chloré, engendré à partir du diméthylphosphite, permet dans les conditions appropriées l'estérification sélective de l'hydroxyle en position 3.

Les esters ainsi préparés et soumis à l'action de l'iodure de sodium donnent ensuite naissance aux phosphates monosodiques correspondants.

Ainsi, la condensation sur la naloxone du cyanoéthyl N,N-diisopropylchlorophosphoramidite au sein du chlorure de méthylène en présence de diisopropyléthylamine conduit au monoester en position 3, qui par action du cyano-2-éthanol et du tétrazole puis oxydation au moyen de l'hydroperoxyde de tert. butyle, génère le phosphate correspondant. Les groupements protecteurs cyanoéthyle sont éliminés par l'ammoniac au sein du méthanol, le sel d'ammonium est ensuite transformé en analogue sodé par passage sur résine échangeuse d'ions.

En ce qui concerne la naltrexone, la séquence utilisée fait appel au pyrophosphate de tétrabenzyle. Ainsi, le phénate de lithium ou de sodium, obtenu par traitement de la naltrexone respectivement avec le disopropylamidure à basse température ou avec l'hydure de sodium à température ambiante, est aisément phosphorylé au moyen du pyrophosphate précédemment mentionné.

L'hydrogénolyse de l'ester formé suivie d'une chromatographie sur résine échangeuse d'ions, permet d'isoler alors le sel disodique recherché.

Le même processus est utilisable avec la naloxone si ce n'est qu'en raison de la double liaison allylique,

la coupure des esters benzyliques est réalisée avec le bromure de triméthylsilyle.

A partir des dérivés monophosphatés de la naloxone et de la naltrexone, il peut être ensuite réalisé la préparation de dérivés triphosphatés au moyen du 1,1′ carbonyldiimidazole, suivi d'un traitement par le pyrophosphate.

Le 6-β-naltrexol-3-phosphate est obtenu par traitement du 6-β-naltrexol avec le tétrabenzyle pyrophosphate dans les mêmes conditions que celles décrites précédemment, le 6-β-naltrexol étant lui-même engendré à partir de la naltrexone selon la littérature (Chatterjie et al., J. Med. Chem., <u>18</u>, 490, 1975).

En ce qui concerne les composés selon l'invention de formule générale I, il est en outre important d'étudier leurs propriétés d'interaction avec les différents tissus de récepteurs des opiacés : affinité et sélectivité.

Par conséquent, afin d'apprécier celles-ci, il a été testé la capacité de quelques dérivés de la naloxone et de la naltrexone à se fixer sur un récepteur spécifique de ces derniers.

Il est connu que la multiplicité des effets engendrés par les analgésiques de type morphine résulte de l'activation simultanée (non sélective) par cette dernière de plusieurs type -μ,δ et k- de récepteurs spécifiques centraux et périphériques dont les propriétés de liaison <u>in vitro</u> et les distributions régionales ont été clairement différenciées. On connait à l'heure actuelle, de nombreux ligands spécifiques des sites opioïdes.

Ainsi, les sites opioïdes de type μ,δ et k co-existent dans le cerveau des mammifères mais en proportions différentes selon l'espèce. Les pourcentages de sites opioïdes de type μ,δ et k sont respectivement 46, 42 et 11 dans le cerveau de rat, 24, 32 et 44 dans le cerveau de cobaye 43, 19 et 38 dans le cerveau de lapin. Néanmoins, certaines "préparations" contiennent un type particulier de site opioïde en proportion très élevée : le cervelet de lapin contient 80 % de sites de type μ, les cellules hybrides neuroblastome x gliome NG 108-15 : 100 % de sites de type δ et le cervelet de cobaye : 85 % de sites de type k.

Il a en outre été mis en évidence une régulation allostérique différentielle de la liaison <u>in vitro</u> des agonistes et des antagonistes non seulement au site de type δ mais aussi aux sites de type μ et k : la fixation d'un agoniste est fortement inhibée en présence d'ions $Na^+$ et de 5′-guanylylimidodiphosphate (GppNHp) alors que, dans ces conditions, la fixation d'un antagoniste ne l'est pas.

Les dérivés de la naloxone et de la naltrexone, objets de la présente invention, se caractérisent également par une affinité apparente satisfaisante pour les sites opioïdes μ et k.

La présente invention se rapporte également aux compositions pharmaceutiques caractérisées en ce qu'elles comportent, à titre de principe actif, au moins un composé selon l'invention.

Selon un mode préféré de l'invention, on utilisera un composé de formule générale I.

Les composés de formule générale I peuvent également être utilisés selon l'invention à titre de prodrogues de la naloxone ou de la naltrexone.

La présente invention se rapporte égalememt aux érythrocytes rescellés contenant au moins un dérivé selon l'invention.

Par érythrocyte rescellé, on entend désigner un érythrocyte qui a subi une lyse puis une reconstitution de la membrane érythrocytaire.

Les exemples donnés ci-dessous à titre non limitatif permettront de mettre en évidence d'autres caractéristiques de la présente invention.

<u>EXEMPLE 1</u> :

**Préparation des dérivés disulfatés de la naloxone et de la naltrexone en positions 3 et 14**

0,30 mmol du chlorhydrate de naloxone (ou naltrexone) sont agités en présence d'un excès de dicyclohexylcarbodiimide (3,13 mmol) dans du diméthylformamide (4 ml). L'ensemble est maintenu à 0°C. Un volume de 0,5 ml d'une solution froide de $H_2SO_4$ (0,2 ml, 3,47 mmol) est ajouté au mélange. Après 1 heure d'agitation à 0°C, le mélange est ajusté à pH9 avec $NH_4OH$ (10 %) et la dicyclohexylurée est éliminée. Après évaporation, le résidu obtenu est dissous dans 1 ml de DMF, ensuite filtré pour éliminer les sels inorganiques. Le dérivé 3,14-disulfate précipite avec de l'acétate d'éthyle. La filtration donne un produit blanc.

Rendement : $R_1$ = $-CH_2-CH=CH_2$ : 79 % ; $R_2$ = 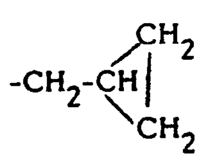 : 86 %

Description des composés :

Pour $R_1$ = $-CH_2-CH=CH_2$ :
IR (KBr) : ν($OSO_3$) 1250 $cm^{-1}$ ; ν(C=O) 1710 $cm^{-1}$.

pour $R_1$ = 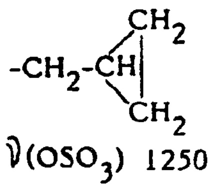

IR (KBr) : ν($OSO_3$) 1250 $cm^{-1}$ ; ν(C=O) 1720 $cm^{-1}$.

**EXEMPLE 2 :**

**Préparation du dérivé monosulfaté de la naloxone et de la naltrexone en position 3**

La diacétylation de la naloxone (ou naltrexone) (0,46 mmol) en présence de 4 ml d'anhydride acétique se fait à reflux pendant une heure. Après évaporation, le résidu est dissous dans du dichlorométhane, et extrait plusieurs fois au moyen d'une solution aqueuse de 5 % NaOH puis avec de l'eau. La phase organique est évaporée, et le produit est purifié par passage sur une colonne de silice (éluant : MeOH-$CH_2Cl_2$ : 1/9).

Rendement : $R_1$ = $-CH_2-CH=CH_2$ : 91 % ; $R_1$ = 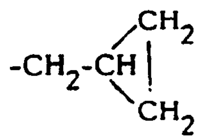 : 86 %

Description des composés :

Pour $R_1$ = $-CH_2-CH=CH_2$ :
IR (KBr) : ν(C=O, 14 acétate) 1730 $cm^{-1}$ ; ν(C=O, 3 acétate) 1760 $cm^{-1}$.

Pour $R_1$ = 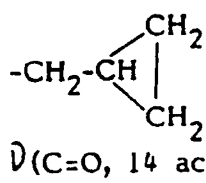 :
IR (KBr) : ν(C=O, 14 acétate) 1720 $cm^{-1}$ ; ν(C=O,3-acétate) 1760 $cm^{-1}$.

0,33 mmol du composé 3,14-acétate sont hydrolysés par action de 12 ml d'une solution aqueuse de $H_2SO_4$ à 4 % pendant 24 heures à température ambiante. Le mélange réactionnel est ajusté à pH8 avec $NH_4OH$ (10 %), et extrait au dichlorométhane. Après séchage et évaporation de la phase organique, le dérivé 14-acétate est purifié sur une colonne de silice (éluant : MeOH-$CH_2Cl_2$ : 1/9).

Rendement : $R_1$ = $-CH_2-CH=CH_2$ : 77 % ; $R_1$ = 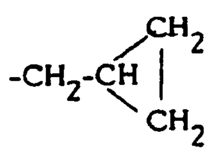 : 79 %

Description des composés :

Pour $R_1$ = $-CH_2-CH=CH_2$ :
IR (KBr) : ν(C=O, 14-acétate) 1730 $cm^{-1}$ ; ν(O-H) 3420 $cm^{-1}$.

Pour $R_1$ = 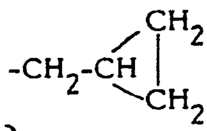

IR (KBr) : ν(C=O, 14-acétate) 1730 $cm^{-1}$ ; ν(O-H) 3420 $cm^{-1}$.

Un mélange de dérivé 14-acétate (0,26 mmol) et de DCCI (3,00 mmol) est agité dans 3 ml de la DMF, auxquels on ajoute ensuite 1 ml d'une solution froide de $H_2SO_4$ (0,029 ml, 0,518 mmol) dans du DMF à 0°C. Après une heure d'agitation, la dicyclohexylurée est éliminée par simple filtration, après avoir basifié le mélange réactionnel avec $NH_4OH$ (10 %) à pH9. Le résidu obtenu, dissous dans 2 ml de DMF, est de nouveau filtré pour éliminer les sels inorganiques. Le produit précipite par action de l'acétate d'éthyle.

Rendement : $R_1$ = $-CH_2-CH=CH_2$ : 63 % ; $R_1$= 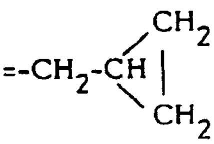 : 50 %

<u>Description des composés</u> :

Pour $R_1$ = $-CH_2-CH=CH_2$ :

IR (KBr) : ν($OSO_3$) 1250 $cm^{-1}$ ; ν(C=O, 14-acétate) 1730 $cm^{-1}$.

Pour $R_1$ = 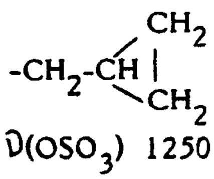

IR (KBr) : ν($OSO_3$) 1250 $cm^{-1}$ ; ν(C=O, 14-acétate) 1730 $cm^{-1}$.

Le passage au dérivé 3-monosulfaté, se fait par traitement de (0,10 mmol) du composé 3-sulfate- 14-acétate par une solution diluée de $NH_4OH$ (20 ml, pH9). L'hydrolyse est maintenue pendant 2 heures à température ambiante sous agitation constante. Après évaporation, le monosulfate dissous dans 1 ml de DMF, précipite en présence de l'acétate d'éthyle.

Rendement : $R_1$ = $-CH_2-CH=CH_2$ : 65 % ; $R_2$ = 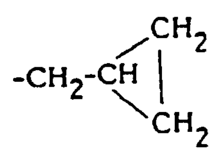 : 75 %.

<u>Description des composés finaux</u>

Pour $R_1$ = $-CH_2-CH=CH_2$ :

IR (KBr) : ν($OSO_3$) 1250 $cm^{-1}$ ; ν(C=O) 1720 $cm^{-1}$.

Pour $R_1$ = 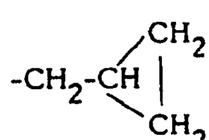 :

IR (KBr) : ν($OSO_3$) 1250 $cm^{-1}$ ; ν(C=O) 1720 $cm^{-1}$.

**EXEMPLE 3 :**

**Préparation des monophosphates monosodiques de la naloxone et de la naltrexone**

A -10°C sous agitation on ajoute goutte à goutte à un mélange de 1 eq de naloxone (ou naltrexone) dissous dans du dichlorométhane et 5 eq de la triéthylamine, 3 eq de $(CH_3O)_2P(O)Cl$ (dilués 20 fois dans le benzène). A la fin de l'addition, le mélange est ramené à température ambiante puis agité 48 heures. Après concentration à sec, on reprend le résidus dans du dichlorométhane. Ensuite on réalise un lavage avec du bicarbonate à 5 % puis rapidement avec de l'eau, pour obtenir le dérivé phosphate diméthylé avec des rendements de :

$R_1$ = $-CH_2-CH=CH_2$ : 83 % ; $R_1$ = 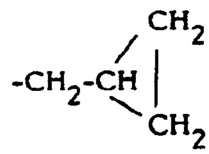 : 85 %

La déméthylation se réalise au moyen d'un excès d'iodure de sodium (5 eq) dans l'acétone, et conduit au monophosphate monosodique.

Rendement : $R_1$ = $-CH_2-CH=CH_2$ : 95% ; $R_1$ = 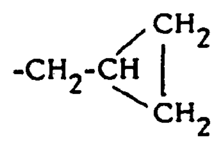 : 96 %

Description des composés :

Pour $R_1$ = $-CH_2-CH=CH_2$ :
IR (KBr) : $\nu$(P=O) 1240 $cm^{-1}$ ; $\nu$(C=O) 1720 $cm^{-1}$.

Pour $R_1$ = 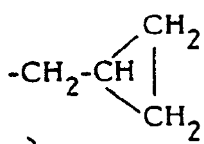 :
IR (KBr) : $\nu$(P=O) 1240 cm-1 ; $\nu$ (C=O) 1740 $cm^{-1}$.

**EXEMPLE 4 :**

**Préparation des dérivés monophosphatés disodiques de la naloxone et de la naltrexone**

1) Phosphorylation par le 2-cyanoéthyl-N,N-diisopropylchlorophosphoramidite I

Le traitement de naloxone (ou naltrexone) (0,55 mmol) par le 2-cyanoéthyl N,N-diisopropylchlorophosphoramidite (0,72 mmol) au sein du dichlorométhane en présence de N-éthyl- N,N-diisopropylamine (1.07 mmol), conduit au monoester correspondant avec un rendement de 99 %. La réaction se fait sous argon à température ambiante pendant 1 heure. Ensuite par action du cyano-2-éthanol (0,55 mmol) et du tétrazole (0.65 mmol) pendant 1 heure, puis oxydation par de l'hydroperoxyde de tert. butyle (0,2 ml) pendant 1 h 20 on obtient le dérivé phosphaté dont les groupements protecteurs cyanoéthyles sont éliminés par l'ammoniac dans du méthanol. On transforme ensuite ce dérivé en phosphate disodique par passage sur résine échangeuse d'ions. Rendement : 55 %.

2) Phosphorylation par le tétrabenzyle pyrophosphate (TBPP)

Deux méthodes sont décrites :

**A)** A une solution à 0°C de naloxone (ou naltrexone) (0,15 mmol) en présence de diisopropylamidure de lithium (0,16 mmol) dans le tétrahydrofurane, on ajoute à -78°C, sous argon, (0,322 mmol) du TBPP. Le mélange est maintenu 1 heure à -78°C, puis ramené à 0°C. On ajoute une solution de bicarbonate saturée à 0°C. Après extraction au dichlorométhane, le produit isolé est purifié sur colonne de silice (éluant : MEOH-$CH_2Cl_2$ : 5/95).

Rendement : $R_1$ = $-CH_2-CH=CH_2$ : 84 % ; $R_1$ = 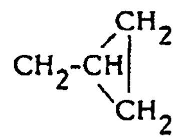 : 84 %

**B)** A une solution de naloxone (ou naltrexone) (1,33 mmol) dans le THF, on ajoute (1,33 mmol) de NaH, sous argon à température ambiante. Après 10 min, le TBPP (1,94 mmol) en solution dans le THF est ensuite additionné. Le mélange est maintenu à température ambiante pendant 18 heures. Le même traitement que celui utilisé dans la méthode **A** permet d'isoler les produits recherchés avec de meilleurs rendements.

Rendement : $R_1$ = $-CH_2-CH=CH_2$ : 90 % ; $R_1$ = 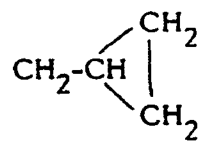 : 95 %

Description des composés :

Pour $R_1$ = $-CH_2-CH=CH_2$ :
IR (KBr) : $\nu$(P=O) 1245 $cm^{-1}$ ; $\nu$(C=O) 1725 $cm^{-1}$.

Pour $R_1$ = 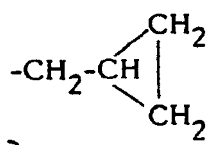 :
IR (KBr) : $\nu$(P=O) 1250 $cm^{-1}$ ; $\nu$(C=O) 1730 $cm^{-1}$.

Déprotection

Dans le cas de la naltrexone dibenzylphosphate, on pratique une hydrogénolyse, $H_2$/Pd dans MeOH, suivie d'une chromatographie sur résine échangeuse d'ions.
Rendement : 84 %.

En raison de la double liaison allylique présente dans la naloxone, la débenzylation se fait par du bromure de triméthylsilyle, suivie d'une purification par chromatographie sur colonne D.E.A.E. Trisacryl,M. On transforme ensuite ce dérivé en phosphate disodique par passage sur une résine échangeuse d'ions.
Rendement : 68 %.

Description des composés :

Pour $R_1$ = $-CH_2-CH=CH_2$ :
IR (KBr) : $\nu$(P=O) 1240 $cm^{-1}$ ; $\nu$(C=O) 1720 $cm^{-1}$.

Pour $R_1$ = 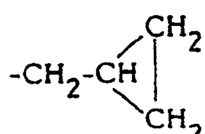 :
IR (KBr) : $\nu$(P=O) 1250 $cm^{-1}$ ; $\nu$(C=O) 1730 $cm^{-1}$.

## EXEMPLE 5 :

**Préparation des dérivés triphosphatés de la naloxone et de la naltrexone.**

Le 3-monophosphate de naloxone (ou naltrexone) (0,24 mmol) est converti en sel de pyridinium par passage sur une colonne échangeuse d'ions DOWEX 50X8 (forme pyridinium). Le traitement de la solution aqueuse ainsi obtenue par la tributylamine (0,48 mmol), suivi de plusieurs co-évaporations à l'aide de la pyridine sèche et de la diméthylformamide, aboutit au sel de bis-tributylammonium du monophosphate correspondant.

Ce sel est ensuite dissous dans la diméthylformamide, dans laquelle on ajoute (1,49 mmol) du 1,1'-carbonyl-diimidazole (CDI). Le mélange réactionnel est maintenu à température ambiante, sous argon, pendant toute une nuit. Après élimination de l'excès de CDI par addition du méthanol (1,98 mmol), on obtient l'ester activé.

Le pyrophosphate tétrasodium décahydraté (1,19 mmol) est transformé en sel de tétra-butylammonium correspondant sous forme de gomme suivant le protocole identique décrit précédemment. Ce dernier, dissous dans la DMF, est additionné à l'ester activé de départ. La réaction est gardée pendant 45 heures, à température ambiante sous argon. Après évaporation, le produit est purifié par chromatographie échangeuse d'ions D.E.A.E.-Trisacryl M ($CH_3COO^-$) éluée avec un gradient continu (0-0,5 M d'une solution aqueuse d'acétate d'ammonium). La fraction ainsi obtenue correspondant au produit pur est lyophilisée. Rendement : 50 %.

Toutes les étapes de cette synthèse ont été contrôlées par chromatographie sur couche mince de silice (éluant : alcool isopropylique/solution $NH_4OH$ 27 %/eau : 6/3/1).

Description des composés :

Pour $R_1$ = $-CH_2-CH=CH_2$ :

IR (KBr) : $\nu$(P=O) 1245 $cm^{-1}$ ; $\nu$(C=O) 1725 $cm^{-1}$.

Pour $R_1$ = 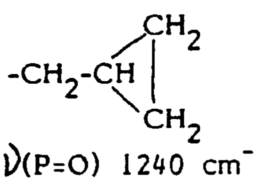 :

IR (KBr) : $\nu$(P=O) 1240 $cm^{-1}$ ; $\nu$(C=O) 1720 $cm^{-1}$.

## EXEMPLE 6 : Préparation du 6-β-naltrexol-3-phosphate,

1) Réduction de la naltrexone en 6-β-naltrexol (Selon Chatterjie et al., J.Med.Chem., 18, 490, 1975).

Une solution de naltrexone.HCl (1mmol) dans 25 ml d'eau est traitée avec un minimum d'une solution de NaOH (320 mg dans 25 ml d'eau) jusqu'à pH alcalin. Le mélange alcalin est traité avec l'acide formamidine sulfinique (4 mmol) dissous dans le reste de la solution NaOH utilisée précédemment. Le mélange réactionnel est agité pendant une heure à 85°C, sous argon.

On ajuste le pH à 9,8 avec quelques d'une solution HCl (6N) et un tampon carbonate-bicarbonate de sodium. Après extraction au dichlorométhane, le produit isolé est purifié sur colonne de silice (éluant : MeOH-$CH_2Cl_2$ :5/95). Rendement : 73%.

Description :

RMN ($CDCl_3$) : 3,45-3,68 ppm (m, H6) ; 4,55 ppm (d, H5, J = 6 Hz) ; 6,55 et 6,70 ppm (2d, H1 et H2, J = 8 Hz).

2) Phosphorylation par le tétrabenzyle pyrophosphate.

A une solution du 6-β-naltrexol (0,52 mmol) dans le THF, on ajoute (0,52 mmol) de NaH, sous argon. Le TBPP (0,79 mmol) en solution dans le THF est ensuite additionné. Le mélange réactionnel est maintenu toute une nuit à température ambiante. Le même traitement que celui mentionné dans la méthode A permet d'isoler le 6-β-naltrexol-3-dibenzylphosphate avec un rendement de 80%.

Description :

RMN ($CDCl_3$) : 3,36-3,49 ppm (m, H6) ; 4,50 ppm (d, H5, J = 6,20 Hz) ; 5,13 et 5,20 ppm (2d, $PhCH_2O$, J = 8,2 Hz) ; 6,57 ppm (d, H1, J = 8 Hz) ; 6,90 ppm (d, H2, J = 8 Hz) et 7,24-7,42 ppm (m, H aromatiques).

3) Déprotection

On pratique une hydrogénolyse, $H_2$/Pd dans MeOH, suivie d'une chromatographie sur une résine échangeuse d'ions. Rendement : 77%.

Description :

IR (KBr) : ν(P=O) 1240 $cm^{-1}$.

Les comportements biologiques de certains composés chimiques ont été ensuite étudiés.

**EXEMPLE 7 :**

Dans un premier temps, on a apprécié les rendements d'internalisation de 5 composés selon l'invention, dérivés de la naloxone ou de la naltrexone. Les résultats sont présentés dans le tableau I.

Tableau I : Résultats d'encapsulation des prodrogues étudiées dans les globules rouges.

| | Rdt Encap | Rdt Hte | Rdt Hb | Rdt Glob |
|---|---|---|---|---|
| Nal.3.MP. (OMe,ONa) | 60% | 73% | 84% | 86% |
| Nalt.3.MP. (OMe,ONa) | 62% | 71% | 81% | 86% |
| Nal.3.MP. (2Na) | 56% | 83% | 86% | 75% |
| Nalt.3.MP. (2Na) | 72% | 75% | 71% | 88% |
| Nal.3;14. disulfate | 71% | 83% | 82% | 89% |
| Nalt.3;14. disulfate | 51% | 74% | 71% | 88% |
| Nal.3.TP. | 37% | 79% | 76% | 84% |
| Nalt.3.TP. | 34% | 90% | 86% | 93% |

Nomenclatures :

Nal. : naloxone
Nalt. : naltrexone
3.MP.(OMe,ONa) : 3 monophosphate (OMe,ONa)
3.MP.(2Na) : 3 monophosphate (2Na)
3.TP. : 3 triphosphate

Rdt encap : rendement d'encapsulation
Rdt Hte : rendement en hématocrite
Rdt Hb : rendement en hémoglobine
Rdt Glob : rendement globulaire

**EXEMPLE 8 :**

Ces composés ont été également testés pour leur absorption membranaire et stabilité érythrocitaire et plasmatique.

Les résultats sont présentés dans le tableau II :

Tableau II : Caractéristiques des molécules étudiées : adsorption membranaire et stabilité érythrocytaire et plasmatique.

| | Adsorption membranaire à Hte 45% | Stabilité dans hémolysat 4°C | Stabilité dans hémolysat 37°C | Stabilité dans sérum frais 4°C | Stabilité dans sérum frais 37°C | GR chargés : Incubation 24h Fuite extra-cellulaire 4°C | GR chargés : Incubation 24h Fuite extra-cellulaire 37°C |
|---|---|---|---|---|---|---|---|
| Nal.3.MP. (OMe,ONa) | 10% | + | + | + | + | 6% | 40% |
| Nalt.3.MP. (OMe,ONa) | 10% | + | + | + | + | 5% | 40% |
| Nal.3.MP. (2Na) | 0% | + | + | + | + | 1% | 12% |
| Nalt.3.MP. (2Na) | 22% | + | + | + | *a | 1% | 12% |
| Nal.3;14. disulfate | 34% | + | + | + | + | 5% | 50% |
| Nalt.3;14. disulfate | 40% | + | + | + | + | 15% | 70% |
| Nal.3.TP. | 22% | + | + | + | *b | 0% | 0% |
| Nalt.3.TP. | 22% | + | + | + | *c | 0% | 0% |

+ : stable.
* : stabilité dans le sérum frais à 37°C au bout de 24h : on ne retrouve que 8 à 12%, 78% et 65% de la quantité de départ dans le sérum respectivement pour (a), (b) et (c).

**EXEMPLE 9** :

Criblage in vitro de dérivés de la naloxone et la naltrexone en tant que ligands opioïdes de type μ ou k.

Animaux

Les animaux utilisés sont des lapins néo-zélandais d'environ 1500 g et des cobayes tricolores d'environ 300 g.

Fraction membranaire brute

La préparation utilisée est une fraction membranaire brute obtenue à partir de tissu frais (cervelet de lapin, cervelet de cobaye) par une méthode standard d'homogénéisation et de centrifugation différentielle. La suspension finale est en tampon tris-HCl (50 mM, pH 7,4) et contient environ 5 mg de protéine par ml.

Radioligand

[15,16(n)-$^3$H] diprenorphine à 25-30 CI/mmol (Amersham International plc, Amersham, Angleterre).

Expériences de compétition

Radioligand (0,3 pmol) de [$^3$H] diprenorphine et fraction membranaire brute (0,25 mg de protéine) sont incubés pendant 1 heure à 25°C dans un volume final de 1,0 ml en tampon Tris-HCl (50 mM, pH 7,4) :

(i) en absence de toute drogue compétitrice ,

(ii) en présence de 12 concentrations croissantes de la nouvelle drogue et,

(iii) en présence de 10 µmol/l de diprenorphine non radioactive.

Le contenu de chaque tube est ensuite rapidement filtré (sous vide) sur disques de fibre de verre (Whatman GF/B) disposés sur un jeu de rampes de filtration Millipore modèle 1225. Les filtres sont rincés avec 2 x 3 ml de tampon froid (O-2°C) puis séchés sous une lampe IR pendant 15 minutes. La radioactivité de chaque filtre, dans 3 ml de cocktail Ready-sol MB (Beckman) est comptée avec un compteur à scintillation liquide Kontron modèle MR 300.

Analyse des données

A partir des paramètres mesurés, il est ensuite calculé les différentes constantes $K_x$ pour les sites considérés.

Dans le tableau III figurent les paramètres caractéristiques de l'inhibition par la naloxone, la naltrexone et par leurs dérivés -3-phosphate, -3-phosphate (OMe), -3-triphosphate, -3-sulfate et -3,14-disulfate de la liaison à l'équilibre de la ($^3$H) diprenorphine (0,3 nmol/l) dans une fraction membranaire brute de cervelet de lapin (sites opioides de type µ) et dans une fraction membranaire brute de cervelet de cobaye (sites opioides de type k).

Ces valeurs ont été calculées à partir des valeurs mesurées des concentrations CI.50 de drogue inhibant de 50 % la liaison à l'équilibre de (3H) diprenorphine utilisée à la concentration fixe de 0,3 nM.

La valeur de K est d'autant plus faible que l'affinité du composé chimique pour le site de fixation est grande.

On peut ainsi noter que les dérivés 3-phosphates ont conservé une excellente affinité apparente pour les sites opioïdes µ et k. La naloxone 3-phosphate semble même avoir une activité légèrement plus élevée que la naloxone pour les deux types de sites opioïdes.

Tableau III : Propriétés pharmacologiques

nmol/l

| produit | (code) | $I_{50\mu}$ | $K_{I\mu}$ | $I_{50\kappa}$ | $K_{I\kappa}$ |
|---|---|---|---|---|---|
| naloxone | (SLN) | 5.2 ± 0.9 (2) | 1.3 | 18 ± 3 (2) | 4.5 |
| naloxone-3-phosphate | (SLP3) | 3.5 ± 0.3 (2) | 0.9 | 4.8 ± 1 (3) | 1.2 |
| naloxone-3-phosphate(OMe) | (SLP1) | 1,200 ± 130 (2) | 260 | 2,500 ± 370 (3) | 615 |
| naloxone-3-triphopshate | (SLP5) | 21 ± 2 (3) | 5,2 | 106 ± 15 (3) | 27 |
| naloxone-3-sulfate | (SLS3) | 4,300 ± 390 (4) | 1,070 | 28,500 ± 4,700 (4) | 7,100 |
| naloxone-3,14-disulfate | (SLS1) | 20,800 ± 3,600 (3) | 5,200 | > 100,000 (3) | > 25,000 |
| | | | | | |
| naltrexone | (SLNT) | 1.2 ± 0.2 (3) | 0.3 | 4.9 ± 0.7 (2) | 1.2 |
| naltrexone-3-phosphate | (SLP4) | 22 ± 2 (5) | 5.6 | 30 ± 3 (4) | 7.5 |
| naltrexone-3-phosphate(OMe) | (SLP2) | 130 ± 9 (5) | 33 | 270 ± 35 (4) | 68 |
| naltrexone-3-triphosphate | (SLP6) | 5.2 ± 0.4 (3) | 1.3 | 15 ± 1.4 (3) | 3.8 |
| naltrexone-3-sulfate | (SLS4) | 1,540 ± 110 (3) | 385 | 2,200 ± 270 (3) | 550 |
| naltrexone-3,14-disulfate | (SLS2) | 7,850 ± 1,300 (3) | 1,960 | 39,000 ± 5,500 (4) | 9,700 |

$I_{50}$ : concentration inhibitrice 50

KI : constante de dissociation apparente du composé testé

## Revendications

1. Dérivé destiné à l'encapsulation dans les érythrocytes d'un composé biologiquement actif hydrophobe caractérisé en ce qu'il est constitué dudit composé biologiquement actif couplé chimiquement à un groupement chimique à caractère hydrophile, par un élément de liaison qui est clivé par les enzymes lysosomiales et/ou plasmatiques mais qui est stable dans les érythrocytes.

2. Dérivé selon la revendication 1, caractérisé en ce que le groupement chimique à caractère hydrophile est une chaîne contenant un ou plusieurs sucres et/ou un ou plusieurs amino-acides.

3. Dérivé selon la revendication 1, caractérisé en ce que le groupement chimique à caractère hydrophile est un radical sulfate, phosphate ou polyphosphate.

4. Dérivé selon la revendication 1 ou 3, caractérisé en ce qu'il est représenté par la formule I

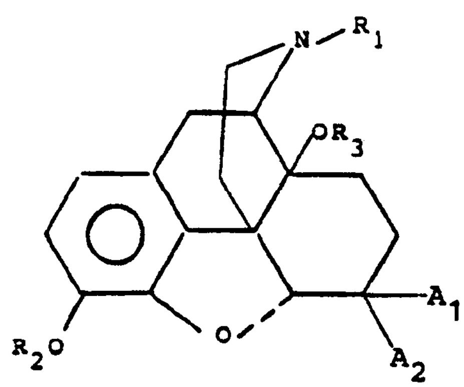

dans laquelle :

- soit **$A_1$** représente le groupement OH et $A_2$ est l'atome d'hydrogène, soit $A_1$ et $A_2$ ensemble avec l'atome de carbone auxquels ils sont rattachés forment un groupement carbonyle

$$(\rangle C{=}O),$$

- **$R_1$** représente un groupement $-CH_2-CH=CH_2$ ou

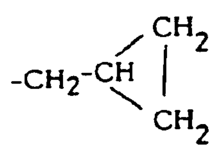

- **$OR_2$** représente un groupement sulfate ou un groupement phosphate ou polyphosphate de formule II :

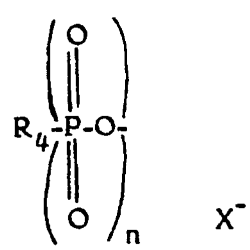

(II)

dans laquelle :

. $R_4$ est choisi parmi un alkoxy en $C_{1-3}$, OH et ses sels alcalins,

. X un cation alcalin et de préférence $Na^+$,

. n représente un entier de 1 à 3, et

– **$OR_3$** représente OH ou un groupement sulfate à la conditions que si **$R_1$** représente $-CH_2-CH=CH_2$ et **A** un groupement CHOH, $OR_2$ et/ou **$OR_3$** sont différents d'un groupement sulfate.

**5.** Dérivé selon la revendication 4, caractérisé en ce qu'il correspond à la formule I dans laquelle $OR_3$ représente un groupement OH, et $OR_2$ un groupement phosphate ou polyphosphate de formule II.

**6.** Dérivé selon la revendication 4 ou 5 caractérisés en ce qu'il présente en outre une affinité apparente satisfaisante pour les sites opioïdes μ et k.

**7.** Composé de formule I :

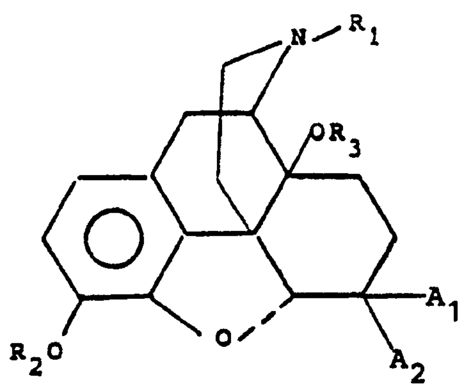

(I)

dans laquelle :

– soit **$A_1$** représente le groupement OH et $A_2$ est l'atome d'hydrogène, soit $A_1$ et $A_2$ ensemble avec l'atome de carbone auxquels ils sont rattachés forment un groupement carbonyle (C=O),

– **$R_1$** représente un groupement $-CH_2-CH=CH_2$ ou

$CH_2$

$-CH_2-CH$

$CH_2$

– **$OR_2$** représente un groupement sulfate ou un groupement phosphate ou polyphosphate de formule II :

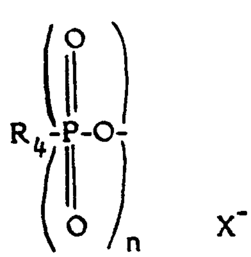

(II)

dans laquelle :

. $R_4$ est choisi parmi un alkoxy en $C_{1-3}$, OH et ses sels alcalins,

. X un cation alcalin et de préférence $Na^+$,

. n représente un entier de 1 à 3, et

– **$OR_3$** représente OH ou un groupement sulfate à la conditions que si **$R_1$** représente $-CH_2-CH=CH_2$ et

**A** un groupement CHOH, $OR_2$ et/ou $\mathbf{OR_3}$ sont différents d'un groupement sulfate.

**8.** Composé selon la revendication 7, caractérisé en ce qu'il correspond à la formule I dans laquelle $OR_3$ représente un groupement OH, et $OR_2$ un groupement phosphate ou polyphosphate de formule II.

**9.** Composé selon la revendication 7 ou 8, caractérisé en ce qu'il présente en outre une affinité apparente satisfaisante pour les sites opioïdes μ et k.

**10.** Composition pharmaceutique caractérisée en ce qu'elle contient à titre de principe actif au moins un dérivé selon l'une des revendications 1 à 6.

**11.** Erythrocyte rescellé, caractérisé en ce qu'il contient au moins un dérivé selon l'une des revendications 1 à 6.